# FASCICULE DE BREVET EUROPEEN

(11) **EP 2 022 510 B1**
(45) Date de publication et mention de la délivrance du brevet: **16.06.2010**
(21) Numéro de dépôt: 08161592.4
(22) Date de dépôt: 31.07.2008
(51) Int. Cl.: A61L 9/12

(54) **Dispositif de parfumage d'ambiance**
Vorrichtung zur Raumparfümierung
Ambient fragrance device

(30) Priorité: 01.08.2007 FR 0705628
(43) Date de publication de la demande: 11.02.2009
(73) Titulaire: Jean, Marcel, 06250 Mougins (FR)
(72) Inventeur: Jean, Marcel, 06250 Mougins (FR)
(74) Mandataire: Roman, Alexis

(56) Documents cités:
- EP-A- 1 561 477
- EP-A- 1 759 899
- WO-A-00/12143
- WO-A-2006/110869
- FR-A- 2 764 806

## Description

### Domaine technique de l'invention.

La présente invention a pour objet un dispositif de parfumage d'ambiance permettant de choisir entre au moins deux fragrances différentes, ou de les mélanger pour en obtenir une nouvelle à volonté en libre choix.

Il se rapporte d'une manière générale au domaine industriel et commercial des articles destinés à assurer un parfumage de haute qualité en diffusant régulièrement dans l'atmosphère un parfum composé de substances volatiles complexes, dans tous les lieux comportant un système d'aération ou de climatisation, que ce soit dans un local ou dans un véhicule, le dispositif pouvant également être associé à tout appareil bureautique ou autre disposant d'un système de ventilation.

### État de la technique.

Il existe à ce jour un grand nombre de dispositifs permettant de diffuser des parfums ou des déodorants, ou tout autre substance volatile destinée à améliorer le confort de l'environnement en particulier pour une utilisation dans les automobiles, par exemple en se fixant sur un aérateur, de manière à exploiter la ventilation pour accélérer à la demande la diffusion des produits actifs en utilisant le système de réglage du débit d'air.

Le brevet N° FR 2.764.806 du même inventeur décrit un dispositif constitué d'un boîtier contenant des granulés de résine polymère du type polyéther-ester amide imprégnés d'une composition parfumée complexe ou de tout autre produit actif destiné à l'amélioration du confort de l'environnement, et étant aménagé en trois compartiments isolés, conçus pour une mise en service en trois périodes successives d'égales importances, le fond de chacun de ces compartiments comportant des ouvertures par lesquelles s'effectue la diffusion du produit parfumé et étant recouvert par une étiquette auto-adhésive munie d'un onglet d'arrachage obturant les ouvertures correspondantes.

Ce dispositif, de même que la plupart des produits similaires existants, ne permet pas de doser la quantité de produit diffusé et n'est pas conçu pour pouvoir fermer les compartiments en cas de non-utilisation.

On connaît par le document brevet EP 1.561.477 (MISAL AREXON), un dispositif de parfumage d'ambiance pour véhicule comportant un boîtier équipé de compartiments à diffusion contrôlable indépendamment, lesdits compartiments étant séparés par des cloisons étanches, chacun desdits compartiments contenant des granulés parfumés. Le boîtier est formé d'une embase et d'un couvercle. Le fond de l'embase et le couvercle sont pourvus d'ouvertures par lesquelles s'effectue la diffusion du parfum hors des compartiments. En pratique, le flux d'air rentre par les ouvertures de l'embase et ressort chargé de parfum par les ouvertures du couvercle. Le boîtier est monté mobile en rotation dans un support équipé d'orifices disposés en vis-à-vis des ouvertures de l'embase. La mise en rotation du boîtier dans le support permet d'obturer partiellement ou totalement les ouvertures de façon à pouvoir activer indépendamment chaque compartiment.

On connaît par le document brevet WO 03/097114 du même inventeur, un élément de parfumage d'ambiance en résine polymère du type polyétherester-amide imprégné d'un produit actif volatil, ledit élément comportant un décor obtenu par transfert au cours du moulage de la pièce, l'injection et le transfert s'effectuant en une seule opération.

On connaît encore par le document brevet WO 00/12143 (AROMA TECHNOLOGY LIMITED) un dispositif complexe comprenant un corps et une cartouche en forme de disque adaptée pour tourner autour de son axe de rotation et comportant une pluralité de compartiments. Chaque compartiment contient un support parfumé. Le corps renferme un élément permettant de générer un flux d'air vers un compartiment présélectionné en réponse à un signal provenant d'un module de commande d'ordinateur, d'un microprocesseur, d'un système optique ou d'un mécanisme de synchronisation. Le corps comporte également un élément permettant de soumettre provisoirement le support parfumé du compartiment au flux d'air de sorte qu'une odeur se dégage de la cartouche et soit entraînée dans ce flux d'air.

On connaît encore par le document brevet WO 03/028775 (PANKHURST) un disperseur de parfum comprenant un seul ventilateur qui fait passer l'air par un parfum simple ou double contenu dans une mèche.

On connaît encore par le document brevet WO 2007/048178 (INTELLECTUAL PROPERTY DEVELOPMENT CORPORATION) un distributeur permettant de distribuer au moins des première et deuxième compositions stockées dans celui-ci, le distributeur comprenant un mécanisme complexe de distribution réglable permettant de distribuer la première composition à un premier débit et la deuxième composition à un deuxième débit. Le mécanisme de distribution est composé d'un premier mécanisme de distribution réglable pour distribuer la première composition au premier débit et d'un deuxième mécanisme de distribution réglable pour distribuer la deuxième composition au deuxième débit.

L'invention, qui constitue un perfectionnement du dispositif décrit dans le brevet N° FR 2.764.806, a pour but de remédier à ces inconvénients grâce à la possibilité de contrôler la diffusion de chaque compartiment indépendamment, chacun de ceux-ci pouvant, à la demande, être fermés ou ouverts totalement ou partiellement.

Un autre objectif de l'invention est d'utiliser un mécanisme de distribution de parfums réglable, plus simple et plus précis que ceux connus de l'art antérieur.

Le dispositif selon l'invention permet en outre de créer de nouvelles fragrances en mélangeant des parfums différents et en faisant varier le dosage de la diffusion de chacun d'eux.

### Divulgation de l'invention.

La solution proposée par l'invention est un dispositif de parfumage d'ambiance du type décrit dans le brevet N° FR 2.764.806, c'est-à-dire comportant un boîtier équipé d'au moins deux compartiments à diffusion contrôlable indépendamment, lesdits compartiments étant séparés par des cloisons étanches, chacun desdits compartiments contenant des granulés parfumés, ledit boîtier étant formé d'une embase et d'un couvercle. Le présent dispositif est remarquable en ce que le fond de l'embase est pourvu d'ouvertures en forme de portions de fentes circulaires concentriques par lesquelles s'effectue la diffusion du parfum hors desdits compartiments, chacune desdites ouvertures débouchant dans un desdits compartiments, ces ouvertures étant obturées partiellement ou totalement au moyen d'obturateurs rotatifs indépendants, tournant autour d'un pivot solidaire de ladite embase. En manoeuvrant les obturateurs, il est ainsi possible de libérer ou d'obturer très simplement les ouvertures et de contrôler la diffusion de chaque compartiment indépendamment.

Les obturateurs sont pourvus chacun d'un levier dépassant sur les côtés du boîtier et permettant de les manoeuvrer et de montrer en face avant le niveau d'ouverture.

Les ouvertures sont réparties chacune en secteurs circulaires, les obturateurs étant eux-mêmes composés de secteurs circulaires déterminés pour recouvrir entièrement lesdites ouvertures, ou être disposés entre ces ouvertures en les dégageant totalement, ou occuper une position intermédiaire.

Les obturateurs comportent avantageusement un joint sur leur périphérie de manière à obturer les ouvertures lorsque lesdits obturateurs sont en position fermée. Dans une variante de réalisation, les obturateurs sont ajustés de manière à être plaqués contre les ouvertures et les obturer de manière étanche lorsque que lesdits obturateurs sont en position fermée.

Le boîtier comprend préférentiellement deux compartiments comportant chacun un élément latéral de fermeture amovible permettant l'introduction aisée des granulés.

Selon un mode particulier de réalisation, l'embase et le couvercle sont solidaires, moulés en une seule pièce et articulés entre eux par une charnière pour permettre l'ouverture et la fermeture du boîtier.

Le moulage de l'embase et du couvercle peut être réalisé en bi-injection pour obtenir une pièce transparente et décorée, avec ou sans fenêtre, dans la même opération d'injection.

Dans une variante de réalisation, le boîtier est réalisé en polypropylène transparent moulé et revêtu d'un décor réalisé par film transfert au moyen du procédé connu sous le nom de "transfert in-mold", soit avec un décor déroulé en continu dans le moule, soit avec pose "IML" d'une image dans chaque empreinte du moule.

Les fentes constituant les ouvertures ont préférentiellement une largeur 10 % à 20 % inférieure au diamètre des granulés parfumés avant augmentation de leur volume par incorporation de parfum.

Chaque compartiment contient avantageusement des granulés ayant un parfum différent.

L'embase est avantageusement équipée à l'arrière d'une pince, pouvant être amovible et interchangeable, agencée pour permettre la fixation du boîtier aussi bien sur un aérateur de voiture que sur une sortie d'air de climatiseur dans un local, ou sur la ventilation d'un appareil de bureautique.

Préférentiellement, la pince est montée sur un support sphérique situé à l'arrière de l'embase, ladite pince étant constituée d'une rotule se montant de manière amovible dans ledit support sphérique, et de trois ailettes dont l'une est opposé aux deux autres.

Avantageusement, le couvercle comporte une ou plusieurs fenêtres transparentes permettant de voir le contenu des compartiments et les granulés sont de couleurs différentes de façon à constituer des codes couleurs permettant d'identifier visuellement et simplement chaque parfum.

Un autre aspect de l'invention consiste à utiliser le dispositif conforme à l'une des caractéristiques précédentes pour reproduire le procédé connu en Parfumerie sous la désignation de « base de Schiff » et consistant à combiner deux senteurs pour en réaliser une troisième qui n'est pas la simple addition des deux autres, ladite utilisation consistant à :
- disposer dans chacun des compartiments des granulés de résine polymère du type polyéther-ester amide imprégnés de compositions parfumées, la senteur étant différente dans chacun desdits compartiments,
- ouvrir totalement ou partiellement chacune des ouvertures,
- faire pénétrer un flux d'air non traversant dans chacun des compartiments, de sorte que ledit flux d'air ressorte desdits compartiments chargé des différents senteurs mélangées pour former une nouvelle senteur.

### Description des figures.

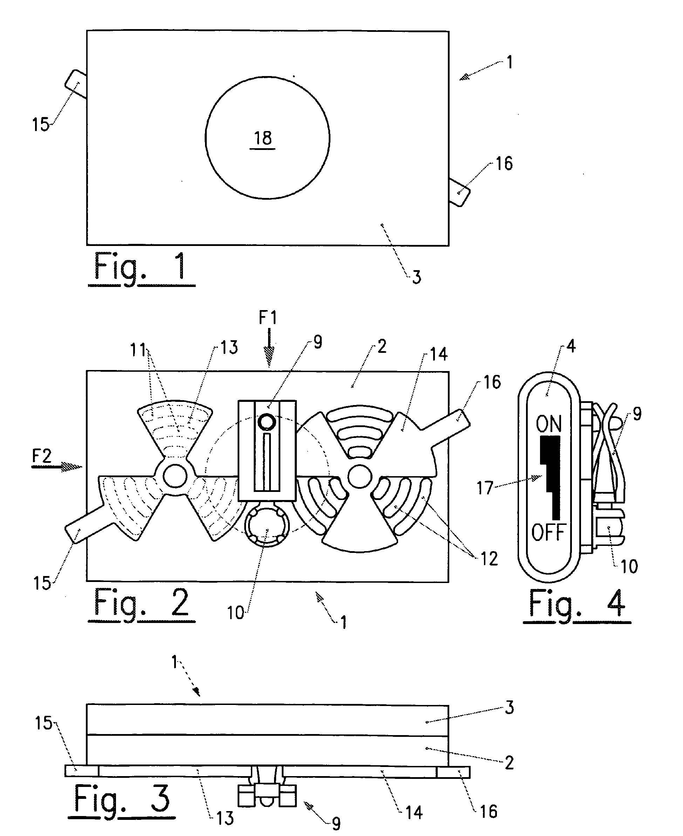

D'autres avantages et caractéristiques de l'invention apparaîtront mieux à la lecture de la description d'un mode de réalisation préféré qui va suivre, en référence aux dessins annexés, réalisés à titre d'exemples indicatifs et non limitatifs et sur lesquels :
- la figure, les figures 1 et 2 représentent le dispositif selon l'invention vu respectivement de devant et de l'arrière,
- la figure 3 est une vue de dessus suivant la flèche F1 de la figure 2,
- la figure 4 représente dans les mêmes conditions le dispositif vu de côté, suivant la flèche F2 de la figure 2,
- la figure 5 est une vue éclatée montrant en perspective axonométrique les différents composants du dispositif
- les figures 6 et 7 représentent, également en perspective axonométrique, une variante du dispositif à couvercle articulé vu en position ouverte, respectivement de l'intérieur et de l'extérieur
- et la figure 8 est une vue agrandie d'une pince articulée de fixation du boîtier.

### Modes de réalisation de l'invention.

Le dispositif objet de l'invention permet de choisir entre au moins deux fragrances différentes, ou de les mélanger pour en obtenir une nouvelle à volonté en libre choix. Il est destiné à diffuser dans l'atmosphère un parfum composé de substances volatiles complexes, dans tous les lieux comportant un système d'aération ou de climatisation, que ce soit dans un local ou dans un véhicule, ou associé à tout appareil disposant d'un système de ventilation.

Le dispositif, figures 1 à 5, est constitué d'un boîtier 1 réalisé de préférence en matière synthétique moulée telle que le polypropylène, avantageusement mono-matière pour favoriser le recyclage, et formé d'une embase 2, d'un couvercle 3 et de deux éléments latéraux 4, 5 de fermeture amovibles permettant l'introduction de granulés imprégnés de compositions volatiles. L'utilisation des deux éléments latéraux 4, 5 simplifie la conception du boîtier 1 et permet un remplissage aisé des compartiments 6, 7.

L'embase 2 et le couvercle 3 sont fixés l'un à l'autre de manière étanche. On pourra par exemple prévoir sur la périphérie de l'embase 2 et/ou du couvercle 3, un joint assurant cette étanchéité.

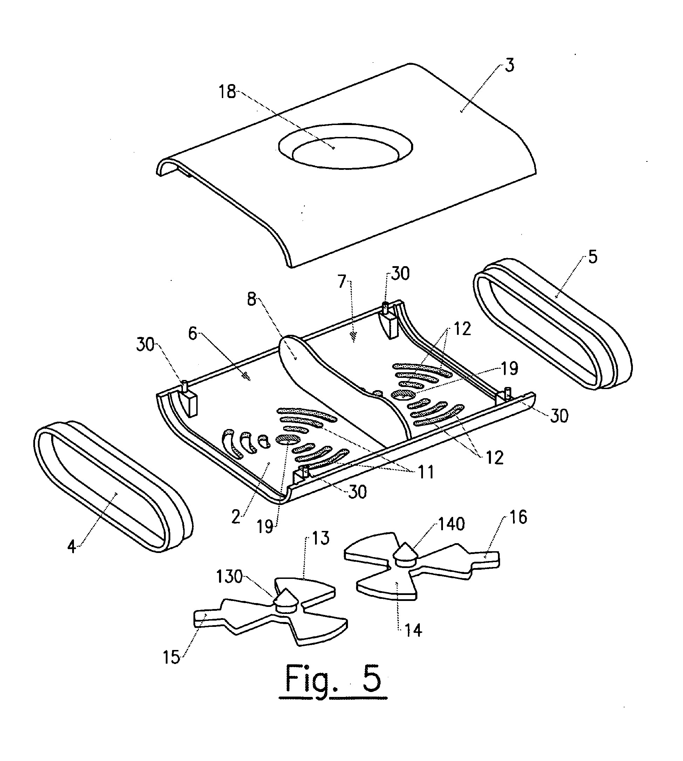

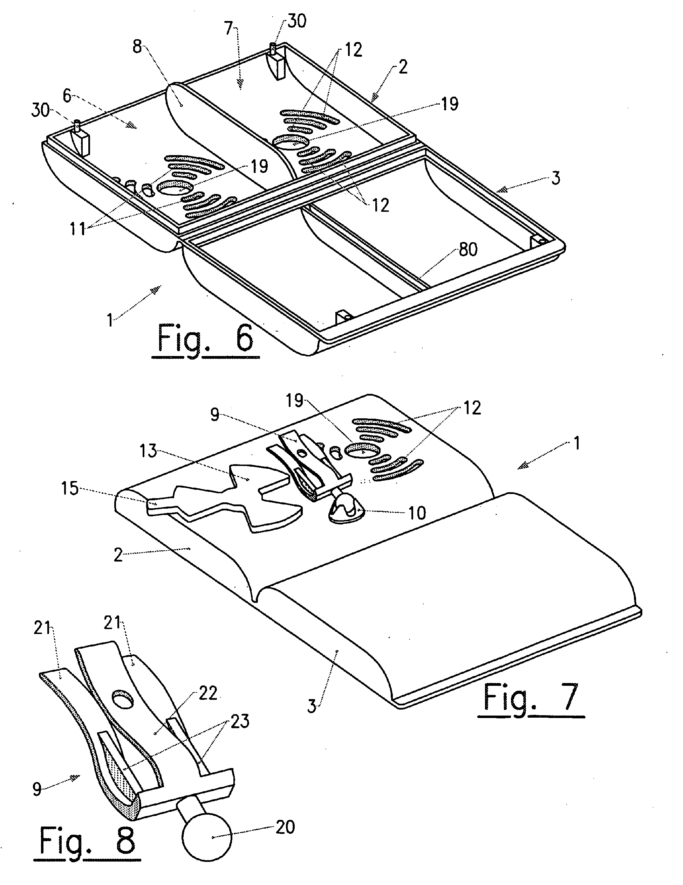

En se rapportant aux figures 5 et 6, il est avantageux de sécuriser la fermeture du boîtier 1 en utilisant des ergots 30 à rupture ou d'autres plots équivalents, de façon à ce qu'un enfant ne puisse plus rouvrir ledit boîtier lorsqu'il est fermé.

Le boîtier 1 comporte deux compartiments 6, 7 séparés par une cloison 8 interne solidaire de l'embase 2, située entre les deux éléments latéraux 4, 5 et formant étanchéité avec le couvercle 3. En se rapportant à la figure 6, pour réaliser l'étanchéité totale entre la cloison 8 et le couvercle 3, on prévoit avantageusement sur la face interne de ce dernier un logement 80 dans lequel s'emboîtera de manière étanche ladite cloison lors de la fixation dudit couvercle sur l'embase 2. Le logement 80 pourra ou non être équipé d'un joint d'étanchéité.

L'embase 2 est pourvue à l'arrière d'un moyen de fixation sur les aérateurs consistant en une pince 9 agencée pour permettre la fixation du boîtier 1 aussi bien sur un aérateur de voiture que sur une sortie d'air de climatiseur dans un local, ou sur la ventilation d'un appareil de bureautique. La pince 9 est fixe ou amovible et interchangeable, montée sur un support sphérique 10 situé à l'arrière de l'embase 2 et solidaire de cette dernière, permettant de l'orienter dans le sens désiré. Cette disposition permet, grâce à un simple changement de pince, d'utiliser le diffuseur dans toutes les situations et dans toutes les orientations.

Cette pince pourra notamment être constituée d'une rotule 20 se montant de manière amovible dans le support sphérique 10, et de trois ailettes 21, 22 dont l'une est opposée aux deux autres (figures 7, 8). En pratique, l'ailette centrale 22 pénètre au milieu des deux autres 21 de manière à former la pince. Pour des considérations de recyclage, la pince 9 est avantageusement réalisée dans la même matière que le boîtier 1, préférentiellement en polypropylène, pour obtenir un ensemble mono-matière. De ce fait, il est avantageux de prévoire sur chaque ailettes 21, 22 des renforts 23 permettant de compenser la faible mémoire de forme de cette matière (figures 8). Ce type de pince permet de s'adapter à toutes les situations verticales ou horizontales inclinées ou non avec des ailettes minces ou épaisses, c'est à dire concrètement aussi bien sur un aérateur de voiture, que sur une sortie d'air de climatiseur dans un local, ou sur la ventilation d'un appareil de bureautique.

Le fond de l'embase 2 est pourvu d'ouvertures 11, 12 en forme de portions de fentes circulaires concentriques par lesquelles s'effectue la diffusion du produit parfumé hors des compartiments 6, 7, ces ouvertures pouvant être obstruées partiellement ou totalement au moyen de deux obturateurs 13, 14 rotatifs indépendants, tournant autour d'un pivot solidaire de l'embase 2 et pourvus chacun d'un levier 15, 16 dépassant sur les côtés du boîtier 1 et permettant de les manoeuvrer et de montrer en face avant le niveau d'ouverture.

Des échelles graduées 17 seront disposées sur les côtés 4 ou en face avant du boîtier 1 pour permettre à l'utilisateur de choisir, pour chaque obturateur 13, 14 une position intégralement fermée, une position ouverte et toutes les positions intermédiaires de façon à pouvoir soit diffuser l'un des parfums de son choix au volume désiré, soit mélanger à doses contrôlées la diffusion des deux différents parfums pour créer une fragrance nouvelle différente des deux senteurs initiales (figure 4). L'utilisateur a donc à sa disposition un réglage indépendant permettant de réguler le flux d'air pénétrant dans chaque compartiment ainsi que la désorption des granulés parfumés, pour obtenir au final un dosage séparé.

Les ouvertures 11, 12 seront par exemple réparties chacune en un à trois secteurs circulaires, les obturateurs 13, 14 étant eux-mêmes composés de un à trois secteurs circulaires déterminés pour pouvoir soit recouvrir entièrement les ouvertures, soit être disposés entre ces ouvertures en les dégageant totalement, soit encore occuper une position intermédiaire. Les fentes constituant les ouvertures 11, 12 seront déterminées de façon telle que l'aération soit suffisante et que les granulés imprégnés de parfum soient toujours retenus, même après une diminution sensible de leur volume, et ceci afin de garantir une sécurité enfant. En pratique, les fentes auront une largeur comprise entre 1.5 mm et 2 mm, soit 10 % à 20 % inférieure au diamètre des granulés parfumés avant augmentation de leur volume par incorporation de parfum.

Les constituants du boîtier 1, ainsi que les obturateurs 13, 14 sont agencés, par tous moyens appropriés, pour que les compartiments 6, 7 soient étanches lorsque lesdits obturateurs sont fermés. En pratique, les obturateurs 13, 14 peuvent comporter un joint sur leur périphérie de manière à obturer les ouvertures 11, 12 lorsque lesdits obturateurs sont en position fermée. De manière équivalente, les obturateurs 13, 14 peuvent être simplement ajustés de manière à être plaqués contre les ouvertures 11, 12 et les obturer de manière étanche lorsque qu'ils sont en position fermée.

Le couvercle 3, dépourvu d'ouvertures, comportera avantageusement une ou plusieurs fenêtres transparentes 18 permettant de voir le contenu des compartiments 6, 7.

Le boîtier 1 peut également être constitué d'une embase 2 et d'un couvercle 3 solidaires, moulés en une seule pièce, et articulés entre eux par une charnière pour permettre l'ouverture et la fermeture dudit boîtier (figure 6). Le moulage de l'embase 2 et du couvercle 3 pourra être réalisé en bi-injection pour obtenir une pièce transparente ou partiellement transparente et décorée avec ou sans fenêtre dans la même opération d'injection.

Le boîtier 1 pourra aussi être réalisé en polypropylène transparent moulé et sera dans ce cas avantageusement revêtu d'un décor réalisé par film transfert au moyen du procédé connu sous le nom de "transfert in-mold", soit avec un décor déroulé en continu dans le moule, soit avec pose "IML" (abréviation de l'expression anglaise In-Mold-Label) d'une image dans chaque empreinte du moule. De plus, les obturateurs 13, 14 seront de préférence agencés pour pouvoir se clipser dans des trous centraux 19 circulaires de l'embase 2, ces trous ayant une taille adaptée au remplissage des perles parfumantes dans chaque compartiment, afin que ce remplissage puisse être automatisé (figure 7). En se rapportant à la figure 5, les obturateurs 13, 14 sont équipés d'un plot, respectivement 130, 140, venant s'emboîter par clipsage ou autre dans les trous centraux 19.

Les deux compartiments 6, 7 sont conçus pour recevoir de préférence des granulés de résine polymère du type polyéther-ester amide imprégnés de compositions parfumées différentes et complexes. Ce type de résine, connue sous la désignation PEBAX®, permet une diffusion régulière et homogène dans le temps de la composition parfumée en phase vapeur. Toutefois, n'importe quel autre type de granulés parfumés imprégnés de compositions volatiles et de manière générale tout autre produit actif destiné à l'amélioration du confort de l'environnement, peut être utilisé.

Les granulés pourront être de couleurs différentes de façon à constituer des codes couleurs permettant d'identifier visuellement et simplement chaque parfum.

Le dispositif permettra donc à l'utilisateur de choisir à volonté l'un ou l'autre des arômes et senteurs, en total libre choix, à sa volonté, selon son inspiration ou les souhaits de ses passagers.

De plus, l'utilisation de perles solides dans ce dispositif déjà décrit dans le brevet mentionné ci-dessus élimine tout risque de fuites, de mélange intempestif ou non désiré de parfum, ce qui n'a pas pu être réalisé jusqu'à présent avec les systèmes comme les sprays, tampons imprégnés, gel, diffuseurs à mèche, etc.

En utilisant une réalisation de l'invention avec deux compartiments, on offrira à l'utilisateur la possibilité de créer une troisième senteur totalement différente des deux premières. Il sera ainsi possible de reproduire le procédé couramment utilisé par les parfumeurs pour leurs créations appelé "Base de Schiff", composition très connue en parfumerie utilisée notamment pour la création des accords floraux et permettant d'obtenir une note nouvelle totalement différente qui n'est pas la simple addition des deux composants.

Pour mémoire une des premières réalisations connue de l'homme de l'art vers le début du vingtième siècle est le mélange de l'hydroxycitronnellal et de l'anthranylate de méthyle qui devient sous cette forme un ingrédient nouveau extrêmement important pour la création des accords de type "FLORAL AGRUME".

Sur le même principe, la présente invention permet à l'utilisateur d'un parfum de combiner deux senteurs pour en réaliser lui même une troisième à sa demande, et si les deux premiers constituants ont été judicieusement choisis au départ, on lui permettra ainsi d'avoir un troisième choix qu'aucun procédé connu en parfumage ne permet d'obtenir à ce jour. Si le dispositif objet de l'invention comporte plus de deux compartiments, il est possible d'avoir une multitude de combinaisons possibles permettant de combiner deux ou plus de deux senteurs pour en réaliser d'autres différentes.

La présente invention apporte donc au domaine du parfumage d'ambiance la possibilité de s'agrandir de façon totalement nouvelle. Le procédé permettra au parfumage de reproduire ce que l'on connaît bien dans le domaine du goût : 1er goût + 2ème goût = 3ème goût que l'on retrouve de façons multiples en gastronomie parmi des exemples célèbres : le Cappuccino, la Pêche Melba, et bien d'autres encore. Les différents parfums sont préférentiellement choisis en fonction de leur complémentarité, par exemple, pêche/vanille, café/chocolat, etc.

Pour ce faire, il suffit de disposer préalablement dans chacun des compartiments 6, 7 des granulés de résine polymère du type polyéther-ester amide (PEBAX®) imprégnés de compositions parfumées, la senteur étant différente dans chacun desdits compartiments. On préfère utiliser des granulés en PEBAX® car ils se prêtent mieux à ce procédé relatif à la "base de Schiff". Il suffit alors que l'utilisateur ouvre totalement ou partiellement les ouvertures 11, 12 de chaque compartiments 6, 7 et fasse pénétrer un flux d'air non traversant dans ces derniers, de sorte que ledit flux d'air ressorte desdits compartiments chargé des différents senteurs mélangées pour former une nouvelle senteur.

L'utilisateur a également la possibilité de diffuser selon son envie un seul des parfums, en ouvrant totalement ou partiellement un compartiment et en fermant le ou les autres.

En résumé, les deux compositions parfumées imprégnant les granulés contenus dans les compartiments 6, 7 sont telles qu'elles permettent au consommateur de reproduire lui-même le procédé connu en Parfumerie sous la désignation de "base de Schiff", et ainsi d'obtenir une note nouvelle totalement différente qui n'est pas la simple addition des deux composants.

### Documents connus de l'état de la technique.

- FR 2.764.806 (JEAN).
- EP 1.561.477 (MISAL AREXON).
- WO 03/097114 (JEAN).
- WO 00/12143 (AROMA TECHNOLOGY LIMITED).
- WO 03/028775 (PANKHURST).
- WO 2007/048178 (INTELLECTUAL PROPERTY DEVELOPMENT CORPORATION).

## Revendications

1. Dispositif de parfumage d'ambiance comportant un boîtier (1) équipé d'au moins deux compartiments (6, 7) à diffusion contrôlable indépendamment, lesdits compartiments étant séparés par des cloisons étanches (8), ledit boîtier étant formé d'une embase (2) et d'un couvercle (3), le fond de l'embase (2) est pourvu d'ouvertures (11, 12) en forme de portions de fentes circulaires concentriques par lesquelles s'effectue la diffusion du parfum hors desdits compartiments, chacune desdites ouvertures débouchant dans un desdits compartiments, ces ouvertures étant obturées partiellement ou totalement au moyen d'obturateurs (13, 14) rotatifs indépendants, tournant autour d'un pivot solidaire de ladite embase, **se caractérisant par le fait que** chacun desdits compartiments contient des granulés parfumés, lesdites ouvertures (11, 12) étant réparties chacune en secteurs circulaires, lesdits obturateurs étant eux-mêmes composés de secteurs circulaires déterminés pour recouvrir entièrement lesdites ouvertures, ou être disposés entre ces ouvertures en les dégageant totalement, ou occuper une position intermédiaire,
et **par le fait que** les obturateurs (13, 14) sont pourvus chacun d'un levier (15, 16) dépassant sur les côtés du boîtier (1) et permettant de les manoeuvrer et de montrer en face avant le niveau d'ouverture, des échelles graduées (17) étant disposées sur les côtés (4) ou en face avant dudit boîtier pour permettre à l'utilisateur de choisir, pour chaque obturateur (13, 14) une position intégralement fermée, une position ouverte et toutes les positions intermédiaires de façon à pouvoir soit diffuser l'un des parfums de son choix au volume désiré, soit mélanger à doses contrôlées la diffusion des deux différents parfums pour créer une fragrance nouvelle différente des deux senteurs initiales.

2. Dispositif selon la revendication 1, dans lequel, les obturateurs (13, 14) comportent un joint sur leur périphérie de manière à obturer les ouvertures (11, 12) lorsque lesdits obturateurs sont en position fermée.

3. Dispositif selon la revendication 1, dans lequel les obturateurs (13, 14) sont ajustés de manière à être plaqués contre les ouvertures (11, 12) et les obturer de manière étanche lorsque que lesdits obturateurs sont en position fermée.

4. Dispositif selon l'une des revendications précédentes, dans lequel les granulés parfumés sont des granulés de résine polymère du type polyéther-ester amide imprégnés de compositions parfumées différentes.

5. Dispositif selon l'une des revendications précédentes, dans lequel chaque compartiment (6, 7) contient des granulés ayant un parfum différent.

6. Dispositif selon l'une des revendications précédentes, dans lequel le boîtier (1) comprend deux compartiments (6, 7) comportant chacun un élément latéral (4, 5) de fermeture amovible permettant l'introduction des granulés.

7. Dispositif selon l'une des revendications précédentes, dans lequel l'embase (2) et le couvercle (3) sont solidaires, moulés en une seule pièce et articulés entre eux par une charnière pour permettre l'ouverture et la fermeture du boîtier (1).

8. Dispositif selon les revendications 7, dans lequel le moulage de l'embase (2) et du couvercle (3) est réalisé en bi-injection pour obtenir une pièce transparente et décorée, avec ou sans fenêtre, dans la même opération d'injection.

9. Dispositif selon la revendication 7, dans lequel le boîtier (1) est réalisé en polypropylène transparent moulé et revêtu d'un décor réalisé par film transfert au moyen du procédé connu sous le nom de "transfert in-mold", soit avec un décor déroulé en continu dans le moule, soit avec pose "IML" d'une image dans chaque empreinte du moule.

10. Dispositif selon l'une des revendications précédentes, dans lequel les fentes constituant les ouvertures (11, 12) ont une largeur 10 % à 20 % inférieure au diamètre des granulés parfumés avant augmentation de leur volume par incorporation de parfum.

11. Dispositif selon l'une quelconque des revendications précédentes, dans lequel l'embase (2) est équipée à l'arrière d'une pince (9) agencée pour permettre la fixation du boîtier (1) aussi bien sur un aérateur de voiture que sur une sortie d'air de climatiseur dans un local, ou sur la ventilation d'un appareil de bureautique, ladite pince étant montée sur un support sphérique (10) situé à l'arrière de l'embase (2), ladite pince étant constituée d'une rotule (20) se montant de manière amovible dans ledit support sphérique, et de trois ailettes (21, 22) dont l'une est opposée aux deux autres.

12. Dispositif selon la revendication 11, dans lequel la pince (9) est amovible et interchangeable.

13. Dispositif selon l'une quelconque des revendications précédentes, dans lequel le couvercle (3) comporte une ou plusieurs fenêtres transparentes (18) permettant de voir le contenu des compartiments (6, 7).

14. Dispositif selon la revendication 13, dans lequel les granulés sont de couleurs différentes de façon à constituer des codes couleurs permettant d'identifier visuellement et simplement chaque parfum.

15. Utilisation du dispositif conforme à l'une des revendications précédentes pour reproduire le procédé connu en Parfumerie sous la désignation de « base de Schiff » et consistant à combiner deux senteurs pour en réaliser une troisième qui n'est pas la simple addition des deux autres, ladite utilisation consistant à :
- disposer dans chacun des compartiments (6, 7) des granulés de résine polymère du type polyéther-ester amide imprégnés de compositions parfumées, la senteur étant différente dans chacun desdits compartiments,
- ouvrir totalement ou partiellement chacune des ouvertures (11, 12),
- faire pénétrer un flux d'air non traversant dans chacun des compartiments (6, 7), de sorte que ledit flux d'air ressorte desdits compartiments chargé des différents senteurs mélangées pour former une nouvelle senteur.

## Claims

1. An ambiance odorizer consisting of a box (1) with at least two controllable diffusion compartments (6, 7), the compartments being separated by tight partitions (8), the said box consisting of a base (2) and a cover (3), the bottom of base (2) being equipped with openings (11, 12) in the form of concentrically arranged slits through which fragrance is diffused outside the said compartments, each of the said slits opening into the said compartments, these slits being partly or totally closed by their own rotary closing devices (13, 14) that rotate around a pivot joined to the said base, **characterised in that** each of the said compartments contains scented pellets, said openings (11, 12) being each one arranged in segments of a circle, the said closing devices themselves consisting of circular segments designed to entirely cover the said openings or be arranged between these openings so that they are fully open, or occupy an intermediate position,
and **in that** closing devices (13, 14) are each one equipped with a lever (15, 16) extending beyond the sides of box (1) so that the user can operate them and show the degree of opening on the front face, graduated scales (17) being arranged on sides (4) or on the front face of the said box so that the user is able to select for each closing device (13, 14) a fully closed position, an open position and any intermediate position to allow either a fragrance of choice to diffuse into a given volume, or to mix in a controlled manner the diffusion of the two different fragrances in order to create a new fragrance different from the two initial fragrances.

2. Device according to claim 1, in which closing devices (13, 14) are equipped with a seal on their periphery that seals openings (11, 12) when the said closing devices are in a closed position.

3. Device according to claim 1, in which closing devices (13, 14) are adjusted so as to be placed tightly against openings (11, 12) and seal them when the said closing devices are closed.

4. Device according to one of the above claims in which scented the pellets are polymer resin pellets of the amide ester-polyether type impregnated with different scented compositions.

5. Device according to one of the above claims in which each compartment (6, 7) contains pellets with a different fragrance.

6. Device according to one of the above claims, in which box (1) has two compartments (6, 7) each one with a lateral removable closing component (4, 5) serving to introduce the pellets.

7. Device according to one of the above claims, in which base (2) and cover (3) are cast as a single part and hinged to allow box (1) to be opened and closed.

8. Device according to claim 7, in which base (2) and cover (3) are made by bi-injection moulding process to obtain a transparent and decorated part, with or without window, using the same injection operation.

9. Device according to claim 7, in which box (1) is made of moulded transparent polypropylene decorated by a transfer film using the process known as "transfer inmould", either with decoration continuously unrolled in the mould, or by the IML process placing an image in each mould impression.

10. Device according to one of the above claims, in which the slits comprising openings (11, 12) are 10% to 20% less wide than the diameter of the scented pellets before their volume is increased by incorporating the scent.

11. Device according to any one of the above claims in which base (2) is equipped with a clip (9) on the back arranged to allow box (1) to be fastened either on a vehicle ventilator or on a room air conditioning outlet, or on office ventilation equipment, the said clip being fitted on a ball support (10) positioned at the rear of base (2), the said clip consisting of a ball joint (20) that fits removable in the said ball support, and three blades (21, 22), one of which acts in opposition to the two others.

12. Device according to claim 11, in which clip (9) is removable and interchangeable.

13. Device according to any one of the above claims, in which cover (3) has one or several transparent windows (18) for viewing the contents of compartments (6, 7).

14. Device according to claim 13, in which the pellets are of different colours so as to constitute colour codes for easy visual identification of each fragrance.

15. Use of the device in accordance with one of the above claims in order to reproduce the process known in the perfume industry as "Schiff base" and consisting in combining two fragrances in order to create a third one which is not the simple addition of the two others, the said use consisting in:
- placing in each compartment (6, 7) pellets of polymer resin of the amide ester polyether type impregnated with scented compositions, the fragrance being different in each of the said compartments,
- total or partial opening of each of opening (11, 12),
- causing a traversing airflow to penetrate each of compartments (6, 7), so that the said airflow exits the said compartments loaded with different fragrances mixed to form a new fragrance.

## Patentansprüche

1. Vorrichtung zur Raumbeduftung bestehend aus einem Gehäuse (1) mit mindestens zwei Abteilen (6, 7) zur voneinander unabhängig gesteuerten Verbreitung von Düften, bei dem die Abteile durch dicht schließende Trennwände (8) voneinander getrennt sind, das Gehäuse aus einem Unterteil (2) und einem Deckel (3) besteht und das Unterteil (2) mit Öffnungen (11, 12) in Form konzentrischer, begrenzt kreisförmiger Spalte versehen ist, durch welche der Duftstoff aus den genannten Abteilen ausströmen kann, wobei jede Öffnung in ein Abteil mündet, die Öffnungen ganz oder teilweise mit Hilfe drehbarer, voneinander unabhängiger Verschlusselemente (13, 14) verschlossen sind, die um eine mit dem Unterteil fest verbundene Achse drehbar sind,
**dadurch gekennzeichnet, dass** jedes Abteil ein aromatisiertes Granulat enthält, dass jede der genannten Öffnungen (11, 12) in kreisförmige Sektoren unterteilt ist, dass auch jedes der Verschlusselemente in kreisförmige Sektoren unterteilt ist, in der Weise, dass die genannten Öffnungen entweder völlig verdeckt sind, oder so zwischen diesen Öffnungen angeordnet werden, dass diese entweder völlig offen sind, oder eine Zwischenstellung einnehmen, sowie **dadurch**,
dass jedes der Verschlusselemente (13, 14) einen Hebel (15, 16) besitzt, der über die Seiten des Gehäuses (1) hinausragt und ihre Betätigung ermöglicht, sowie die Anzeige des Öffnungsgrades auf der Vorderseite, dass auf beiden Seiten (4) oder auf der Vorderseite des Gehäuses Skaleneinteilungen (17) angeordnet sind, anhand derer der Benutzer mit jedem einzelnen Verschlusselement (13, 14) entweder die völlig geschlossene, die völlig offene oder auch alle Zwischenstellungen einstellen kann, so dass er entweder nur einen der Duftstoffe in dem gewünschten Volumen oder auch eine gewünschte Mischung beider Duftstoffe ausströmen lassen kann, um so eine neue, sich von den beiden ursprünglichen Düften unterscheidende Duftnote zu erzeugen.

2. Vorrichtung gemäß Patentanspruch 1, bei dem die Verschlusselemente (13, 14) auf ihrem Umfang eine Dichtung besitzen, um die Öffnungen (11, 12) zu verschließen, wenn sich die Verschlüsse in der geschlossenen Stellung befinden.

3. Vorrichtung gemäß Patentanspruch 1, bei dem die Verschlusselemente (13, 14) so angepasst sind, dass sie sich an die Öffnungen (11, 12) anlegen und sie hermetisch verschließen, wenn sich die Verschlüsse in der geschlossenen Stellung befinden.

4. Vorrichtung gemäß einem der vorstehenden Patentansprüche, bei welcher das aromatisierte Granulat aus einem Polymer-Kunstharz vom Typ Polyesteramid besteht, das mit unterschiedlichen Duftkompositionen aromatisiert ist.

5. Vorrichtung gemäß einem der vorstehenden Patentansprüche, bei welcher beide Abteile (6, 7) Granulat unterschiedlicher Duftnote enthalten.

6. Vorrichtung gemäß einem der vorstehenden Ansprüche, bei welcher das Gehäuse (1) zwei Abteile (6, 7) besitzt, von denen jedes ein seitliches Element (4, 5) besitzt, das einen abnehmbaren Verschluss darstellt und zum Einfüllen des Granulats dient.

7. Vorrichtung gemäß einem der vorstehenden Patentansprüche, bei welcher das Unterteil (2) und der Deckel (3) als einteiliges Bauteil geformt und miteinander durch ein Scharnier drehbar verbunden sind, um das Öffnen und Schließen des Gehäuses (1) zu ermöglichen.

8. Vorrichtung gemäß Patentanspruch 7, bei welcher das Formen des Unterteils (2) und des Deckels (3) durch Zweikomponenten-Spritzgießen erfolgt, um ein transparentes Teil mit Dekor, mit oder ohne Fenster, im gleichen Spritzguss-Arbeitsgang zu erhalten.

9. Vorrichtung gemäß Patentanspruch 7, bei welcher das Gehäuse (1) aus transparentem Polypropylen ein Spritzgussteil ist und entweder mit dem unter dem Namen "Transfert in-mold" bekannten Verfahren mit einer Dekorfolie, oder mit einem kontinuierlich in die Form abgewickelten Dekorfilm, oder mit einem bei jedem Formabdruck im IML-Verfahren eingelegten Bild versehen wird.

10. Vorrichtung gemäß einem der vorstehenden Patentansprüche, bei der die Spalte, welche die Öffnungen (11, 12) bilden, eine um 10% bis 20% geringere Breite als der Durchmesser der Granulatkörner besitzen, bevor diese mit dem Duftstoff aromatisiert wurden.

11. Vorrichtung gemäß einem der vorstehenden Patenansprüche, bei welcher das Unterteil (2) auf seiner Rückseite mit einer Klammer (9) versehen ist, die derart angeordnet ist, dass sie die Befestigung des Gehäuses (1) sowohl an einer Belüftungsdüse eines Fahrzeugs, als auch am Luftaustritt einer Klimaanlage in einem Raum oder an der Lüftung eines Bürogerätes ermöglicht, wobei die Klammer auf einer kugelförmigen Halterung (10) montiert ist, die sich auf der Rückseite des Unterteils (2) befindet, und die Klammer aus einem Kugelgelenk (20) besteht, das lösbar in der genannten kugelförmigen Halterung montiert wird, sowie aus drei Flügeln (21, 22), von denen einer den beiden anderen gegenüber steht.

12. Vorrichtung gemäß Patentanspruch 11, bei welcher die Klammer (9) abnehmbar und austauschbar ist.

13. Vorrichtung gemäß einem der vorstehenden Patenansprüche, bei welcher der Deckel (3) ein oder mehrere transparente Fenster (18) besitzt, durch welche der Inhalt der Abteile (6, 7) zu sehen ist.

14. Vorrichtung gemäß Patentanspruch 13, bei welcher die Granulatkörner verschiedene Farben haben, so dass Farbcodes entstehen, anhand derer jede Duftnote einfach optisch zu erkennen ist.

15. Verwendung der einem der vorstehenden Patentansprüche entsprechenden Vorrichtung zur Ausführung des im Parfümeriegewerbe unter der Bezeichnung "Base de Schiff' bekannten Verfahrens, das darin besteht, zwei Duftnoten zu kombinieren, um eine dritte zu erzeugen, die nicht einfach die Summe der beiden anderen ist, bestehend darin,
- in jedes Abteil (6, 7) mit Duftstoff-Kompositionen getränkte Polymer-Kunstharzkörner vom Typ Polyesteramid zu füllen, wobei die Duftnote in beiden Abteilen unterschiedlich ist,
- beide Öffnungen (11, 12) ganz oder teilweise zu öffnen,
- Luft in jedes der beiden Abteile (6, 7) eintreten, jedoch nicht durchtreten zu lassen, derart, dass der Luftstrom, wenn er aus den Abteilen austritt, mit einer Mischung verschiedener Duftnoten angereichert ist, die eine neue Duftnote bilden.
